# EUROPÄISCHE PATENTANMELDUNG

(11) **EP 2 005 938 A1**
(43) Veröffentlichungstag der Anmeldung: **24.12.2008**
(21) Anmeldenummer: 08009772.8
(22) Anmeldetag: 29.05.2008
(51) Int. Cl.: A61K 8/06, A61K 8/22, A61K 8/37, A61K 8/39, A61K 8/60, A61Q 17/04

(54) **Kosmetische oder dermatologische Zubereitungen mit einem Gehalt an molekularem Sauerstoff, grenzflächenaktiven Glucosederivaten und vorteilhafterweise einer oder mehreren UV-Licht absorbierenden Substanzen**

(30) Priorität: 14.06.2007 DE 102007028025
(71) Anmelder: Beiersdorf AG, 20245 Hamburg (DE)
(72) Erfinder: Bleckmann, Andreas, 22926 Ahrensburg (DE); Treu, Jens, 22844 Norderstedt (DE); Fey, Sven, 22397 Hamburg (DE); Rathsack, Jessica, 21614 Buxtehude (DE); Möllgaard, Svenja Lena, 22301 Hamburg (DE); Peters, Nils, 22965 Todendorf (DE)

(57) **Zusammenfassung**

Zubereitungen mit einem Gehalt an molekularem Sauerstoff und grenzflächenaktiven Glucosederivaten.

## Beschreibung

Die Erfindung umfasst kosmetische oder dermatologische Zubereitung mit einem Gehalt an molekularem Sauerstoff und grenzflächenaktiven Glucosederivaten und vorteilhafterweise einer oder mehreren UV-Licht absorbierenden Substanzen.

Die Stabilität der mit Sauerstoff beladenen Zubereitungen und die Sauerstoffverfügbarkeit aus den Zubereitungen ist verbessert.

Gashaltige kosmetische Zubereitungen sind an sich bekannt und bereits in zahlreichen Patenten beschrieben worden. Als Gase werden beispielsweise Sauerstoff, fluorierte Gase, Kohlendioxid, Luft, Stickstoff und dergleichen verwendet.

Eine Möglichkeit zur Stabilisierung von Gasen in kosmetischen oder dermatologischen Zubereitungen besteht z, B. darin, eine O/W-Emulsion herzustellen, welche anschließend mit Gas beaufschlagt wird. Derartige Formulierungen sind auch als Mousse bekannt (beispielsweise WO 2002-074258-A1). Nachteilig an diesen Zubereitungen des Standes der Technik ist, dass das Gas nur durch den Einsatz von Emulgatoren stabilisiert werden kann. Ferner nachteilig ist, dass das Gas bei der topischen Applikation oder bereits bei Lagerung bei 40 °C oder mehr (beispielsweise im Auto oder am Strand) in die Atmosphäre entweichen kann. Ferner ist die Beladungskapazität derartiger Zubereitungen meistens eher gering, so dass sich physiologische Effekte nach der Applikation nicht einstellen.

Aus dem Stand der Technik sind kosmetische Zubereitungen auf Basis von O/W-Emulsionen bekannt, die Sauerstoff enthalten.

Die WO 02/05754 beschreibt extern applizierbare Zubereitungen, die einen Sauerstoffträger enthalten, der in einer lipoiden Emulsion molekulardispers eingearbeitet ist, sowie deren Verwendung zur externen Behandlung / Prävention von Sauerstoffmangelzuständen der Haut.

Die WO 02/05754 offenbart O/W-Emulsionen, die als einen Sauerstofftransport durch die Lipidschicht der Haut adäquat gewährleisten sollen.

In der US 5851544 wird beschrieben, dass Hautpflegezubereitungen, welche das Hautsauerstoffniveau erhöhen, wünschenswert seien, da der Sauerstoffgehalt in der Haut niedriger sei als in anderen Körperregionen und darüber hinaus mit zunehmendem Alter abnehme. Aufgabe der US 5851544 ist es daher, den Sauerstoffgehalt der Haut langfristig zu steigern bzw. Sauerstoffmangelzustände in der Haut zu beheben.

In der DE 10333710 werden kosmetische, dermatologische oder pharmazeutische Zubereitungen auf Basis von Lipiden und/oder Lipid/Wachs-Gemischen, welche ein Gas bzw. ein Gasgemisch enthalten, beschrieben.

Die DE 10342449, DE 102005011498, DE 102005011457 und DE 102206011459 offenbaren kosmetische Zubereitungen enthaltend molekularen Sauerstoff. Auch hierin werden bevorzugt O/W-Emulsionen beschrieben. Die Herstellung der sauerstoffhaltigen O/W-Emulsionen erfolgt indem eine Vordispergierung des anorganischen Gelbildners und Quellung des Hydrokolloides unter Rühren in der Wasserphase stattfindet. Eine Vereinigung der auf 75 °C aufgeheizten Fettphase mit der auf 70 °C aufgeheizten Wasserphase und Zugabe der partikulären hydrophoben, hydrophobisierten Festkörpersubstanzen unter Rühren. Es erfolgt dann Homogenisierung bei 65 °C und 45 min Rühren unter Vakuum und Kühlung. Die Zugabe der Additive erfolgt bei 30 °C, die Homogenisierung bei 27 °C. Anschließend wird die Begasung der Emulsion bei 7-8 bar mit Sauerstoff durchgeführt.

Die Herstellung sauerstoffhaltiger kosmetischer Zubereitungen und insbesondere der Schritt der Sauerstoffzufuhr erfolgt in allen aufgeführten Druckschriften des Standes der Technik dabei unter Kühlung, bei Raumtemperatur bzw. bei maximal 30°C.

Nachteilig an den bekannten Zubereitungen ist, dass nach Einarbeitung des molekularen Sauerstoffs, die Zubereitungen nicht stabil lagerfähig sind, eine geringe Sauerstoffbeladungskapazität aufweisen und lediglich geringe Sauerstoffverfügbarkeit aufweisen.
- Wünschenswert ist es daher eine kosmetische Zubereitung bereit zu stellen, die molekularen Sauerstoff enthält und die eine für Kosmetika ausreichende Lagerfähigkeit aufweist. Ebenso ist es wünschenswert ein Verfahren zur Herstellung sauerstoffhaltiger Emulsionen zur Verfügung zu haben, mit dem eine kosmetisch anwendbare und lagerfähige Zubereitung herstellbar ist.
-
- Unter kosmetischer Hautpflege ist in erster Linie zu verstehen, daß die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) gestärkt oder wiederhergestellt wird.
-
- Wird diese Funktion gestört, kann es zu verstärkter Resorption toxischer oder allergener Stoffe oder zum Befall von Mikroorganismen und als Folge zu toxischen oder allergischen Hautreaktionen kommen.
-
- Wünschenswert ist es auch Produkte zur Pflege der auf natürliche Weise gealterten Haut, sowie zur Behandlung der Folgeschäden der Lichtalterung zur Verfügung zu stellen.
-
- Produkte zur Pflege gealterter Haut sind an sich bekannt. Sie enthalten z. B. Retinoide (Vitamin A-Säure und/oder deren Derivate) bzw. Vitamin A und/oder dessen Derivate, Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.
-
- Produkte zum Schutz vor schädlichen Oxidationsprozessen in der Haut sind an sich bekannt. Auch Produkte, welche die Sauerstoff-Versorgung der Haut steigern sollen, sind bereits im Stand der Technik beschrieben. Allerdings konnte der Stand der Technik nicht den Weg zur vorliegenden Erfindung weisen.
-
- Bei verstärktem Kontakt polymerer Verdickungsmittel mit Gasen kann es bekanntermaßen zu unangenenhmen Geruchsentwicklungen kommen. Bei begasten Kosmetika ist dies besonders mißlich, da diese Produkte ja allenfalls geruchsfrei, in den meisten Fällen aber durch beigemischtes Parfum mit Wohlgeruch ausgestattet sein sollen.

Diese Aufgaben werden gelöst durch Zubereitungen mit einem Gehalt an molekularem Sauerstoff, ferner enthaltend
(a) einer oder mehreren grenzflächenaktiven Substanzen A, gewählt aus der Gruppe der Glucosederivate, welche sich durch die Strukturformel 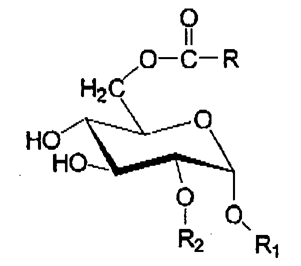 auszeichnen, wobei R einen verzweigten oder unverzweigten Alkylrest mit 1 bis 24 Kohlenstoffatomen darstellt, wobei R₁ entweder ein Wasserstoffatom oder einen verzweigten oder unverzweigten Alkylrest mit 1 bis 24 Kohlenstoffatomen darstellt und wobei R₂ entweder ein Wasserstoffatom oder einen verzweigten oder unverzweigten Acylrest mit 1 bis 24 Kohlenstoffatomen darstellt, enthalten,
(b) einer oder mehreren grenzflächenaktiven Substanzen B, gewählt aus der Gruppe der Substanzen der allgemeinen Strukturformel 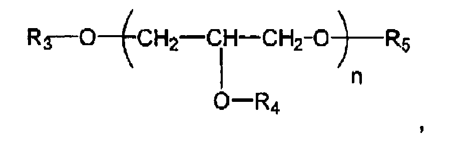 wobei R₃, R₄ und R₅ voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt
   und
(c) vorteilhafterweise einer oder mehreren UV-Licht absorbierenden Substanzen.

Besonders vorteilhaft wird oder werden die grenzflächenaktiven Substanzen A aus der Gruppe Methylglucosemonostearat (Formel wie nachstehend) 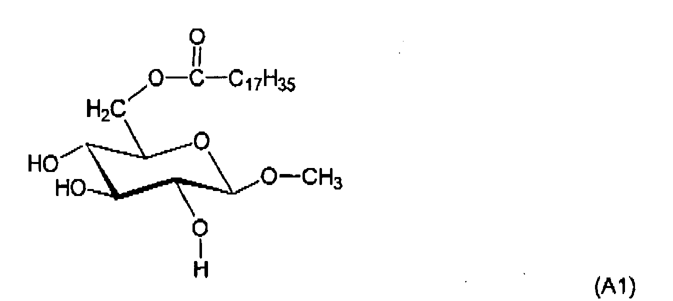 Methylglucosedistearat (Formel wie nachstehend) 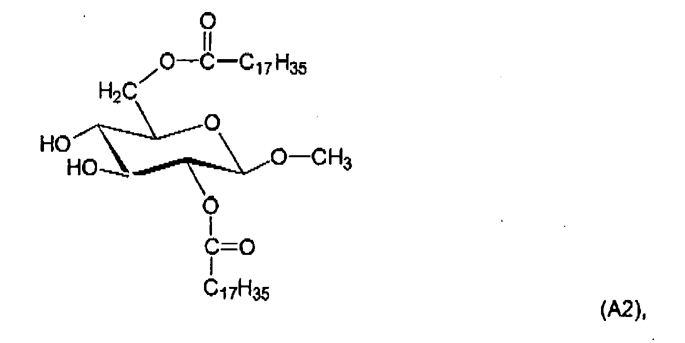 und beliebigen Gemischen daraus, beispielsweise ungefähr äquimolaren Mischungen daraus gewählt, welche auch als Methylglucosesesquistearat bezeichnet werden. Solches Methylglucosesesquistearat ist im Handel beispielsweise unter der Warenbezeichnung Tego® Care PS von der Gesellschaft.Goldschmidt AG erhältlich.

Besonders vorteilhaft wird oder werden die grenzflächenaktiven Substanzen B gewählt aus der Gruppe der Verbindungen, bei welcher n den Wert 3 annimmt und R₃, R₄ und R₅ unabhängig voneinander gewählt werden aus der Gruppe, welche umfaßt: H, verzweigte bzw. unverzweigte, gesättigte bzw. ungesättigte Fettsäurereste mit 14 bis 20 Kohlenstoffatomen, insbesondere die nachfolgend aufgeführten Strukturen: 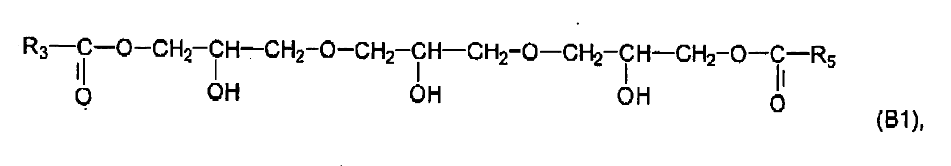 Als erfindungsgemäß bevorzugte Emulgstorkombination hat sich ein ungefähr äquimolekulares Gemisch aus den Verbindungen A2 und B1, wobei in B1 die Reste R₃ und R₅ vorzugsweise beide einen Stearatrest bezeichnen, herausgestellt. Solche Emulgatorkombinationen sind als "Polyglyceryl(3)-Methylglucosedistearat" (PGMS) unter der Warenbezeichnung Tego Care® 450 von der Gesellschaft Goldschmidt AG erhältlich.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 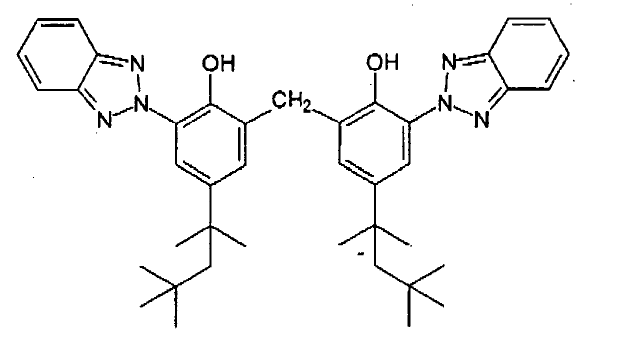 gekennzeichnet ist und unter der Handelsbezeichnung Tinosorb® M bei der CIBA-Chemikalien GmbH erhältlich ist.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure 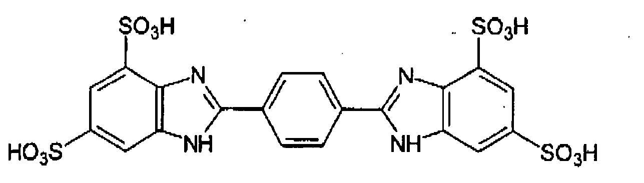 und ihre Salze, besonders die entsprechenden Natrium-, Kalium- oder Triethanotammonium-Salze, insbesondere das Phenylen-1,4-bis-(2-benzimidazyl)-3,3'-5,5'-tetrasulfonsäure-bis-natriumsalz 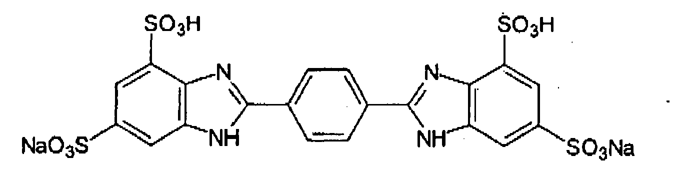 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bomylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenme-thyl-10-sulfonsäure) bezeichnet wird und sich durch die folgende Struktur auszeichnet: 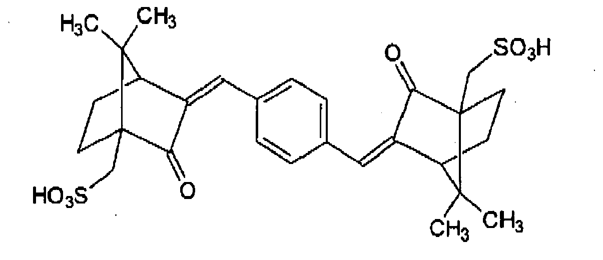 Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 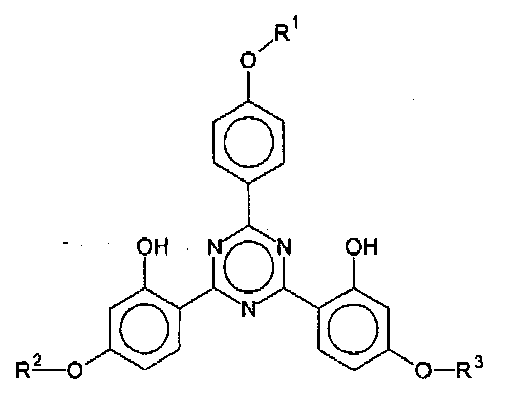 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)2-hydroxy]-phenyl}-6-(4-methoxyphenyl)-1,3,5-triazin (INCl: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCl: Octyl Triazone), welches von der BASF Aktiengesellschaft unter der Warenbezeichnung UVINUL® T 150 vertrieben wird.

Auch andere UV-Filtersubstanzen, welche das Strukturmotiv 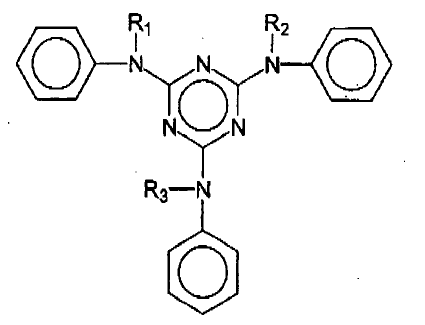 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 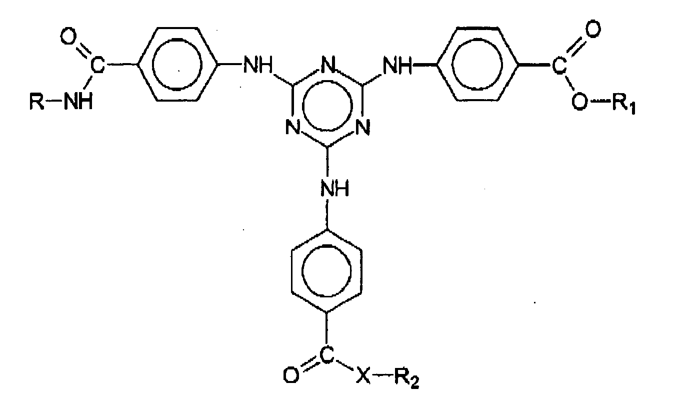 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine-NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 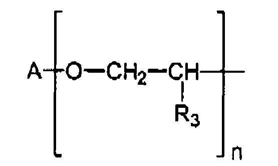 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 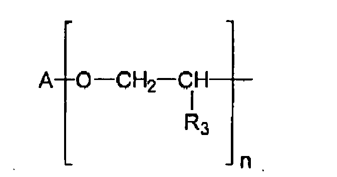 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 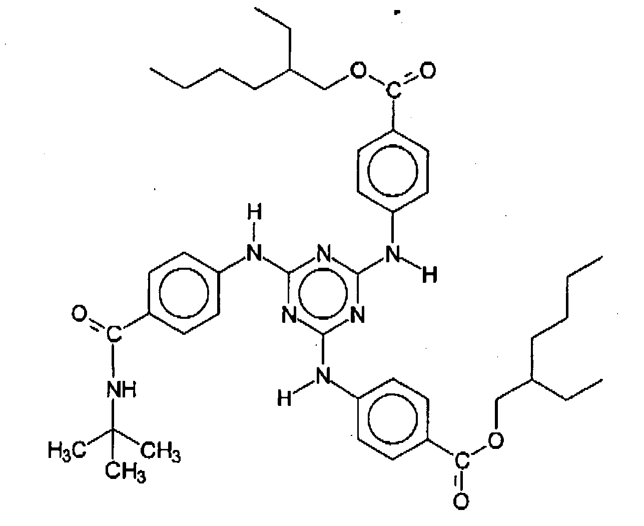 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Auch in der Europäischen Offenlegungsschrift 775 698 werden bevorzugt einzusetzende Bis-Resorcinyltriazinderivate beschrieben, deren chemische Struktur durch die generische Formel 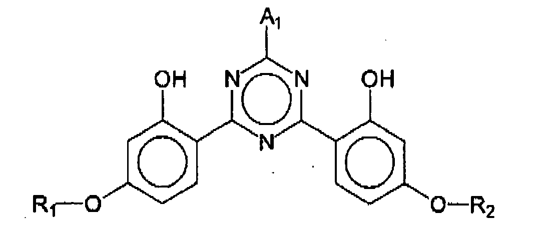 wiedergegeben wird, wobei R₁ , R₂ und A₁ verschiedenste organische Reste repräsentieren.

Vorteilhaft im Sinne der vorliegenden Erfindung sind ferner das 2,4-Bis-{[4-(3-sutfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1, 3, 5-triazin, das 2,4-Bis-{[4-(3-(2-propyloxy)2-hydroxy-propyloxy)-2-hydroxy]-phenyl]-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-(4-methoxyphenyl)-1,3,5-triazin, das 2,4-Bis-{[4-(2"-methylpropenyloxy)-2-hydroxyl-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 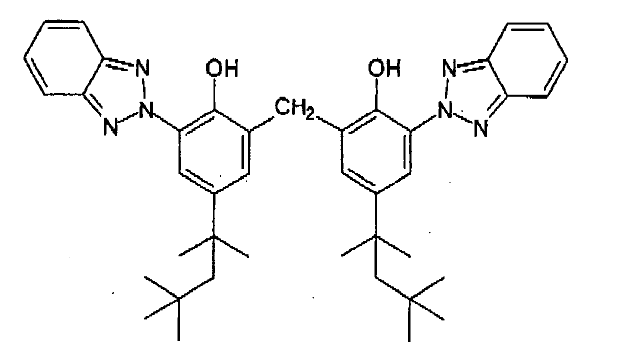 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriezol-2-yl)-4-methyl-6-[2-methyl-3-[1,3,3,3-tetramethyl-1-[(trimethylsiyl)oxy]disiloxanyl]propyl]-phenol (CAS-Nr.: 155633-54-8) mit der INCI-Bezeichnung Drometrizole Trisiloxane, welches durch die chemische Strukturformel. 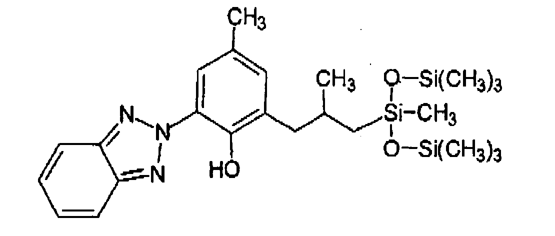 gekennzeichnet ist.

Die UV-B-Filter können öllöslich oder wasserlöslich sein. Vorteilhafte öllösliche UV-B-Filtersubstanzen sind z. B.:

3-Benzylidencampher-Derivate, vorzugsweise 3-(4-Methylbenzyliden)campher, 3-Benzylidencampher;

4-Aminobenzoesäure-Derivate, vorzugsweise 4-(Dimethylamino)-benzaesäure(2-ethylhexyl)ester, 4-(Dimethylamino)benzoesäureamylester;

2,4,6-Trianilino-(p-carbo-2'-ethy)-1'-hexyloxy)-1,3,5-triazin:
Ester der Benzalmalonsäure, vorzugsweise 4-Methoxybenzalmalonsäuredi(2-ethylhexyl)ester:
Ester der Zimtsäure, vorzugsweise 4-Methoxyzimtsäure(2-ethylhexyl)ester, 4-Methoxyzimtsäureisopentylester;
Derivate des Benzophenons, vorzugsweise 2-Hydroxy-4-methoxybenzaphenon, 2-Hydroxy-4-methoxy-4'-methylbenzophenon, 2,2'-Dihydroxy-4-methoxybenzophenon sowie an Polymere gebundene UV-Filter.

Vorteilhafte wasserlösliche UV-B-Filtersubstanzen sind z. B.:
Salze der 2-Phenylbenzimidazol-5-sulfonsäure, wie ihr Natrium-, Kalium- oder ihr Triethanolammonium-Salz, sowie die Sulfonsäure selbst;
Sulfonsäure-Derivate des 3-Benzylidencamphers, wie z. B. 4-(2-Oxo-3-bomylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist und sich durch folgende Struktur auszeichnet:

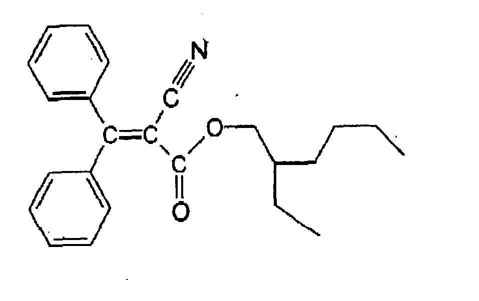

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92120690 beschrieben werden.

Ferner kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Ganz besonders vorteilhaft sind erfindungsgemäße Zubereitungen, dadurch gekennzeichnet, daß die UV-Licht absorbierenden Substanz oder die Substanzen gewählt wird oder werden aus der Gruppe Butylmethoxydibenzoylmethan, Ethylhexylmethoxycinnamate , Octocrylen, Ethylhexylsalicylat, Homosalate, Phenylbenzimidazolsuffonsäure, Dinatriumphenyldibenzimidazoltetrasulfonat, Benzophenon-3, Methylen-bis-benzotriazolyltetramethylbutylphenol, Diethylaminohydroxybenzoylhexylbenzoat, Bis-Ethylhexyloxyphenolmethoxyphenyltriazin.

Vorteilhaft enthalten die erfindungsgemäßen Zubereitungen 5-25 Volumen-°/o Sauerstoff, vorzugsweise 10-20 Volumen-%, insbesondere bevorzugt 12-17 Volumen-.%

Vorteilhaft enthalten die erfindungsgemäßen Zubereitungen 0,1-5 Gew-°/° an Emulgator A, vorzugsweise 0,5-2,5 Gew-% an Emulgator A, insbesondere bevorzugt 1- 2Gew-% an Emulgator A.

Vorteilhaft enthalten die erfindungsgemäßen Zubereitungen 0,1-5 Gew-% an Emulgator B, vorzugsweise 0,5 - 2,5 Gew-% an Emulgator B, insbesondere bevorzugt 1- 2Gew-% an Emulgator B.

Vorteilhaft enthalten die erfindungsgemäßen Zubereitungen 0.1-5 Gew-% an einer oder mehreren UV-Licht absorbierenden Substanzen, vorzugsweise 0,5 - 2,5 Gew-% an an einer oder mehreren UV-Licht absorbierenden Substanzen, insbesondere bevorzugt 1 - 2 Gew-% an einer oder mehreren UV-Licht absorbierenden Substanzen.

Die erfindungsgemäße kosmetische oder dermatologische Zubereitung, umfassend molekularen Sauerstoff ist dadurch charakterisiert, dass sie erhältlich ist durch übliches Mischen und Herstellen einer O/W-Emulsion und anschließendem Begasen der Zubereitung mit gasförmigen Sauerstoff und/oder sauerstoffhaltigen Gasen.

Die erfindungsgemäße kosmetische oder dermatologische Zubereitung auf Basis einer Emulsion, insbesondere einer O/W-Emulsion, umfassend molekularen Sauerstoff ist dadurch charakterisiert, dass sie erhältlich ist durch übliches Mischen und Herstellen einer Emulsion und anschließendem Begasen der Zubereitung mit gasförmigen Sauerstoff und/oder sauerstoffhaltigen Gasen. Dabei wird die Zubereitung bei Raumtemperatur (ca. 20 bis 25 °C) in den Mischer eingespeist und anschließend begast.

Die kosmetischen und dermatologischen Zubereitungen gemäß der Erfindung können kosmetische Hilfsstoffe enthalten, wie sie üblicherweise in solchen Zubereitungen verwendet werden, z. B. Emulgatoren, Pigmente, Konservierungsmittel, Konservierungshelfer, Komplexbildner, Bakterizide, Parfüme, Substanzen zum Steigern des Schäumens, Farbstoffe, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische. Lösungsmittel oder Silikonderivate.

Durch die Verwendung von Sauerstoff bzw. kosmetischer oder topischer dermatologischer Zubereitungen mit einem wirksamen Gehalt an Sauerstoff im Sinne der vorliegenden Erfindung wird die Haut überraschend besser gegen Alterungsprozesse, Struktur- und/oder oxidative Schäden geschützt als dies mit Zubereitungen des Standes der Technik möglich wäre. Ferner wird durch die erfindungsgemäße Verwendung die Wiederherstellung des gesunden Hautzustands im Fall einer bereits bestehenden Störung und Schädigung der normalen Hauteigenschaften begünstigt.

Unter "gestörtem Erscheinungsbild der Haut" sind im Sinne der vorliegenden Erfindung insbesondere die folgenden Phänomene zu verstehen:
■ Falten und/oder Fältchen,
■ Haut- und/oder Gewebeerschlaffung,
■ Störungen der Regeneration der Haut und der Hautanhangsgebilde,
■ Durchblutungsstörungen der Haut,
■ Altersflecken, Pigmentstörungen und sogenannter "uneven skin tone"
■ sensible Haut,
■ Juckreiz,
■ Stressempfindlichkeit der Haut.
■ entzündliche und empfindliche Hautzustände,
■ trockene Hautzustände,
■ atopisches Ekzem,
■ Psoriasis
■ Unregelmäßige bzw. zunehmende Porengröße

Ferner werden bezogen auf die Hautanhangsgebilde (Haare, Nägel) insbesondere die folgenden (Alterungs-) Erscheinungen ("gestörte Erscheinungsbilder der Haare") durch die erfindungsgemäße Verwendung positiv beeinflußt:
- Wachstumsverlangsamung,
- Deformation,
- Spliss,
- Rissigkeit,
- vorzeitiger Verlust,
- Struktur und Farbveränderung.

Die kosmetischen oder dermatologischen Mittel gemäß der Erfindung können vorteilhaft in Form von schäumbaren Zubereitungen vorliegen, welche beispielsweise aus Aerosolbehältern entnommen und dabei aufgeschäumt werden. Erfindungsgemäß vorteilhafte Aerosolbehälter sind Sprühvorrichtungen mit einer Füllung aus den flüssigen bzw. breiartigen Stoffen, die unter dem Druck eines Treibmittels stehen (Druckgas- oder Aerosolpackungen). Derartige Behälter können mit Ventilen sehr unterschiedlicher Bauart ausgestattet sein, die die Entnahme des Inhalts als Schaum ermöglichen.

Die kosmetischen oder dermatologischen Formulierungen im Sinne der vorliegenden Erfindung können wie üblich zusammengesetzt sein und dem kosmetischen oder dermatologischen Lichtschutz, ferner zur Behandlung, Pflege und Reinigung der Haut, der Lippen, sowie von Hautanhangsgebilden (Nägel und/oder Haare) und als Schminkprodukt in der dekorativen Kosmetik dienen.

Ebenso wie Emulsionen von flüssiger und fester Konsistenz als kosmetische Reinigungslotionen bzw. Reinigungscremes Verwendung finden, können auch die Zubereitungen im Sinne der vorliegenden Erfindung "Reinigungsschäume" darstellen, welche beispielsweise zum Entfernen von Schminken und/oder Make-up oder als milder Wasch- und Duschschaum-ggf. auch für unreine Haut - verwendet werden können. Derartige Reinigungsschäume können vorteilhaft ferner als sogenannte "rinse off" Präparate angewendet werden, welche nach der Anwendung von der Haut abgespült werden

Die erfindungsgemäßen kosmetischen und/oder dermatologischen Zubereitungen können auch vorteilhaft in Form eines Schaums zur Pflege des Haars bzw. der Kopfhaut vorliegen, insbesondere eines Schaums zum Einlegen der Haare, eines Schaums, der beim Fönen der Haare verwendet wird, eines Frisier- und Behandlungsschaums.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

| **O/W-Emulsionen** | **1** |
|---|---|
| Polyglyceryl-3 Methyl Glucose Distearat | 2 |
| Avocadoöl | 1 |
| Cyclomethicon | 2,5 |
| Myristyl Myristat | 1 |
| Cetylalkohol | 1,5 |
| Cetylstearylalkohol | 2 |
| Zitronensäure | 0,03 |
| Butylmethoxydibenzoylmethan | 2 |
| Octocrylen | 5 |
| Phenylbenzimidazolsulfonsäure | 1 |
| Titandioxid | 1 |
| EDTA - | 2 |
| BHT | 0,1 |
| 1,2-Hexandiol | 0,2 |
| 1,2-Pentandiol | 0,2 |
| Glycerin | 5 |
| Parfum | 0,3 |
| Carbomer | 0.7 |
| Kreatin | 0,01 |
| Vitamin E Acetat | 1,5 |
| Nyion-12 | 2 |
| Phenoxyethanol | 0,5 |
| Propylparaben | 0,2 |
| Methylparaben | 0,2 |
| Natriumhydroxid | q.s. |
| Wasser | ad 100 |

Die O/W-Emulsion wird nach Herstellung mit 25% Sauerstoff begast.

| **O/W-Emulsionen** | **2** |
|---|---|
| Polyglyceryl-3 Methyl Glucose Distearat | 4 |
| C12-15 Alkylbenzoat | 2 |
| Dimethicon | 1 |
| Dicaprylylcarbonat | 1 |
| Cyclomethicon | 4 |
| Myristyl Myristat | 2 |
| Cetylalkohol | 2 |
| Zitronensäure | 0,05 |
| Butylmethoxydibenzoylmethan | 0,5 |
| Ethylhexyl Methoxycinnamat | 3 |
| EDTA | 1 |
| BHT | 0,05 |
| 1,2-Octandiol | 0,3 |
| Glycerin | 9 |
| Parfum | 0,4. |
| Xanthangummi | 0,4 |
| Vitamin E Acetat | 1 |
| Phenoxyethanol | 0,4 |
| Ethylparaben | 0,1 |
| Propylparaben | 0,1 |
| Methylparaben | 0,2 |
| Natriumhydroxid | 0,2. |
| Wasser | ad 100 |

Die O/W-Emulsion wird nach Herstellung mit 20% Sauerstoff begast.

| **O/W-Emulsionen** | **3** |
|---|---|
| Polyglyceryl-3 Methyl Glucose Distearat | 3 |
| Dimethicon | 4 |
| Butylenglycol Dicaprylat/Dicaprat | 3 |
| Caprylsäure/Caprinsäure Triglyceride | 2 |
| Myristyl Myristat | 0,5 |
| Stearylalkohol | 2 |
| Zitronensäure | 0,01 |
| Bis-Ethylhexyloxyphenolmethoxyphenyltriazin | 1 |
| Ethylhexyltriazon | 1 |
| Octocrylen | 2 |
| Dinatriumphenyldibenzimidazoltetrasulfonat | 4 |
| Zinkoxid | 1 |
| EDTA | 1 |
| Glycerin | 12 |
| Parfum | 0,1 |
| Acrylates/C10-30 Alkyl Acrylate Crosspolymer | 0,4 |
| Panthenol | 1 |
| Ubichinon | 0,01 |
| Vitamin E Acetat | 0,2 |
| Phenoxyethanol | 0,4 |
| Ethylparaben | 0,1 |
| Propylparaben | 0,1 |
| Methylparaben | 0,2 |
| Natriumhydroxid | 0,1. |
| Wasser | ad 100 |

Die O/W-Emulsion wird nach Herstellung mit 15% Sauerstoff begast.

| **O/W-Emulsionen** | **4** |
|---|---|
| Polyglyceryl-3 Methylglucosedistearat | 2,5 |
| C12-15 Alkylbenzoat | 2 |
| Dimethicon | 2 |
| Hydrogenierte Cocoglyceride | 2 |
| Myristyl Myristat | 3 |
| Cetylstearylalkohol | 2 |
| Zitronensäure | 0,05 |
| Butyimethoxydibenzoylmethan | 4 |
| Titandioxid | 1 |
| Zinkoxid | 1 |
| Ethylhexylsalicylat | 2 |
| Homosalat - | 2 |
| EDTA | 1 |
| BHT | 0,1 |
| Ethylhexylglycerin | 2 |
| Glycerin | 10 |
| Parfum | q.s. |
| Acrylates/C10-30 Alkyl Acrylate Crosspolymer | 0,5 |
| Kreatin | 0,1 |
| Ubichinon | 0,01 |
| Tapiocastärke | 1 |
| Phenoxyethanol | 0,5 |
| Propylparaben | 0,2 |
| Methylparaben | 0,2 |
| Natriumhydroxid | 0,15 |
| Wasser | ad 100 |

Die O/W-Emulsion wird nach Herstellung mit 22,5% Sauerstoff begast.

| **O/W-Emulsionen** | **5** |
|---|---|
| Polyglyceryl-3 Methylglucosedistearat | 4 |
| Dicaprylylether | 3 |
| Cyclomethicon | 6 |
| Hydrogenierte Cocoglyceride | 2 |
| Cetyldimeticon | 3 |
| Caprylsäure/Caprinsäure Triglyceride | 2 |
| Myristyl Myristat | 2 |
| Cetylalkohol | 2 |
| Butylmethoxydibenzoylmethan | 0,5 |
| Ethylhexylmethoxycinnamat | 3 |
| EDTA | 1 |
| BHT - | 0,05 |
| 1,2-Octandiol | 0,25 |
| Glycerin | 5 |
| Parfum | 0,3. |
| Xanthangummi | 0,6 |
| Panthenol | 1,5 |
| Vitamin E Acetat | 0,5 |
| Nylon-12 | 2 |
| Phenoxyethanol | 0,4 |
| Ethylparaben | 0,1 |
| Propylparaben | 0,1 |
| Methylparaben | 0,2 |
| Natriumhydroxid | 0,1. |
| Wasser | ad 100 |

Die O/W-Emulsion wird nach Herstellung mit 20% Sauerstoff begast.

| **O/W-Emulsionen** | **6** |
|---|---|
| Polyglyceryl-3 Methylglucosedistearat | 5 |
| Butylenglycol Dicaprylat/Dicaprat | 3 |
| Dicaprylylcarbonat | 2 |
| Cetyldimeticon | 2,5 |
| Myristyl Myristat | 1 |
| Cetylstearylalkohol | 1 |
| Stearylalkohol | 1 |
| Zitronensäure | 0,1 |
| Bis-Ethylhexyloxyphenolmethoxyphenyltriazin | 2 |
| Octocrylen | 5 |
| Dinatriumphenyldibenzimidazoltetrasulfonat | 3 |
| EDTA - | 2 |
| BH-f | 0,1 |
| Methylpropandiol | 2 |
| Glycerin | 15 |
| Parfum | q.s. |
| Carbomer | 0,8 |
| Phenoxyethanol | 0,4 |
| Ethylparaben | 0,1 |
| Propylparaben | 0,1 |
| Methylparaben | 0,2 |
| Natriumhydroxid | 0,1. |
| Wasser | ad 100 |

Die O/W-Emulsion wird nach Herstellung mit 17 % Sauerstoff begast.

| **O/W-Emulsionen** | **7** |
|---|---|
| Polyglyceryl-3 Methylglucosedistearat | 3 |
| C12-15 Alkylbenzoat | 2 |
| Dimethicon | 4 |
| Cyclomethicon | 5 |
| Myristylmyristat | 3 |
| Butylmethoxydibenzoylmethan | 2 |
| Ethylhexylmethoxycinnamat | 2 |
| Ethylhexyltriazon | 2 |
| Ethythexylsalicylat | 2 |
| EDTA | 1 |
| BHT | 0.1 |
| 1,2-Pentandiol | 0,25 |
| Glycerin | 12 |
| Parfum | q.s. |
| Carbomer | 0.6 |
| Folsäure | 0,01 |
| Silica | 1 |
| Phenoxyethanol | 0,5 |
| Propylparaben | 0,2 |
| Methylparaben | 0,2 |
| Natriumhydroxid | 0,15 |
| Wasser | ad 100 |

Die O/W-Emulsion wird nach Herstellung mit 8% Sauerstoff begast.

| **O/W-Emulsionen** | **8** |
|---|---|
| Polyglyceryl-3 Methylglucosedisiearat | 2 |
| Cyclomethicon | 2 |
| Hydrogenierte Cocosfettsäureglyceride | 2 |
| Cetyldimeticon | 3 |
| Caprylsäure/Caprinsäuretriglyceride | 1 |
| Myristylmyristat | 4 |
| Cetylalkohol | 1 |
| Stearylalkohol | 1 |
| Zitronensäure | 0,1 |
| Bis-Ethylhexyloxyphenolmethoxyphenyltriazin | 2 |
| Octocrylen | 1 |
| Titandioxid | 2 |
| 1,2-Hexandial | 2 |
| Glycerin | 4 |
| Parfum | 0,4 |
| Cl 77492 + Cl 77491 + Cl 77499 | 0,1 |
| Cl 77891 | 2 |
| Vitamin E Acetat | 1 |
| Silica + Cl 77891 + Cl 77491 | 1 |
| Phenoxyethanol | 0,4 |
| Ethylparaben | 0,1 |
| Propylparaben | 0,1 |
| Methylparaben | 0,2 |
| Natriumhydroxid | 0,2. |
| Wasser | ad 100 |

Die O/W-Emulsion wird nach Herstellung mit 18 % Sauerstoff begast.

| **O/W-Emulsionen** | **9** |
|---|---|
| Polyglyceryl-3 Methylglucosedistearat | 1 |
| C12-15 Alkylbenzoat | 4 |
| Hydrogenierte Cocosfettsäureglyceride | 1 |
| Myristylmyristat | 4 |
| Cetylalkohol | 1 |
| Cetylstearylalkohol | 1 |
| Stearylalkohol | 1 |
| Butylmethoxydibenzoylmethan | 4 |
| Octocrylen | 4 |
| Phenylbenzimidazolsulfonsäure | 2 |
| BHT | 0,2 |
| Glycerin - | 6,5 |
| Parfum | q.s. |
| Xanthangummi | 0,8 |
| DHA | 1 |
| Cl 77891 + Mica + Silika | 1 |
| Phenoxyethanol | 0,4 |
| Ethylparaben | 0,1 |
| Propylparaben | 0,1 |
| Methylparaben | 0,2 |
| Natriumhydroxid | 0,1. |
| Wasser | ad 100 |

Die O/W-Emulsion wird nach Herstellung mit 15% Sauerstoff begast.

## Patentansprüche

1. Zubereitungen mit einem Gehalt an molekularem Sauerstoff, ferner enthaltend Zubereitungen mit einem Gehalt an molekularem Sauerstoff, ferner enthaltend
(a) einer oder mehreren grenzflächenaktiven Substanzen A, gewählt aus der Gruppe der Glucosederivate, welche sich durch die Strukturformel 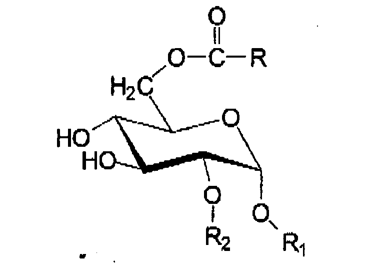 auszeichnen, wobei R einen verzweigten oder unverzweigten Alkylrest mit 1 bis 24 Kohlenstoffatomen darstellt, wobei R₁ entweder ein Wasserstoffatom oder einen verzweigten oder unverzweigten Alkylrest mit 1 bis 24 Kohlenstoffatomen darstellt und wobei R₂ entweder ein Wasserstoffatom oder einen verzweigten oder unverzweigten Acylrest mit 1 bis 24 Kohlenstoffatomen darstellt, enthalten,
(b) einer oder mehreren grenzflächenaktiven Substanzen B, gewählt aus der Gruppe der Substanzen der allgemeinen Strukturformel 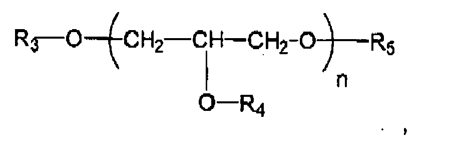 wobei R₃, R₄ und R₅ voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt, und
(c) vorteilhafterweise einer oder mehreren UV-Licht absorbierenden Substanzen.

2. Zubereitungen nach Anspruch 1, in Form von O/W- oder W/O-Emulsionen vorliegend.

3. Zubereitungen nach Anspruch 1, enthaltend 5 - 25 Volumen-% Sauerstoff, vorzugsweise 10 - 20 Volumen-%, insbesondere bevorzugt 12 - 17 Volumen-.%

4. Zubereitungen nach Anspruch 1, enthaltend 0,1 - 5 Gew-% an Emulgator A, vorzugsweise 0,5 - 2,5 Gew-% an Emulgator A, insbesondere bevorzugt 1 - 2Gew-% an Emulgator A.

5. Zubereitungen nach Anspruch 1, enthaltend 0,1 - 5 Gew-% an Emulgator B, vorzugsweise 0,5 - 2,5 Gew-% an Emulgator B, insbesondere bevorzugt 1- 2Gew-% an Emulgator B.

6. Zubereitungen nach einem der vorstehenden Ansprüche, **dadurch gekennzeichnet, daß** A und B in Form eines annähernd äqumolaren Gemisches (Polyglyceryl(3)-Methylglucosedistearat).

7. Zubereitungen nach einem der vorstehenden Ansprüche, enthaltend 0,1 - 5 Gew-% an einer oder mehreren UV-Licht absorbierenden Substanzen, vorzugsweise 0,5 - 2,5 Gew-% an an einer oder mehreren UV-Licht absorbierenden Substanzen, insbesondere bevorzugt 1-2 Gew-% an einer oder mehreren UV-Licht absorbierenden Substanzen.

8. Zubereitungen nach einem der vorstehenden Ansprüche, **dadurch gekennzeichnet, daß** die UV-Licht absorbierenden Substanz oder die Substanzen gewählt wird oder werden aus der Gruppe Butylmethoxydibenzoylmethan, Ethylhexylmethoxycinnamate, Octocrylen, Ethylhexylsalicylat, Homosalate, Phenylbenzimidazolsulfonsäure, Dinatriumphenyldibenzimidazoltetrasulfonat, Benzophenon-3, Methylen-bis-benzotriazolyltetramethylbutylphenol, Diethylaminohydroxybenzoylhexylbenzoat, Bis-Ethylhexyloxyphenalmethoxyphenyltriazin.
